# EUROPEAN PATENT SPECIFICATION

(11) **EP 1 655 603 B1**
(45) Date of publication and mention of the grant of the patent: **13.07.2011**
(21) Application number: 05023423.6
(22) Date of filing: 26.10.2005
(51) Int. Cl.: G01N 33/493

(54) **Urine sugar level measuring apparatus**
Vorrichtung zur Messung des Harnzuckerspiegels
Appareil de mesure de niveau de sucre dans urine

(30) Priority: 04.11.2004 JP 2004320066; 04.08.2005 JP 2005226068
(43) Date of publication of application: 10.05.2006
(73) Proprietor: Tanita Corporation, Tokyo (JP)
(72) Inventor: Ito, Narushi c/o TANITA CORPORATION, Tokyo (JP); Ohashi, Akio c/o TANITA CORPORATION, Tokyo (JP)
(74) Representative: Grünecker, Kinkeldey, Stockmair & Schwanhäusser Anwaltssozietät

(56) References cited:
- US-A- 5 420 108
- US-A1- 2003 208 113

## Description

### BACKGROUND OF THE INVENTION

### (i) Field of the Invention

This invention relates to a urine sugar meter which measures a high blood sugar level in a body before and after meal.

### (ii) Description of the Related Art

As typified by diabetes, the concentration of sugar in the blood or urine becomes high as a body metabolic function with respect to sugar lowers. To treat or prevent diabetes, the body metabolic function with respect to sugar has been examined by measuring a blood sugar level or urine sugar level after passage of a given time after meal by means of a blood sugar meter which measures the concentration of sugar (blood sugar level) in the blood sampled from a subject at the time of its sampling (for example, Patent Publications 1 and 2) or a urine sugar meter which measures the concentration of sugar (urine sugar level) in the urine discharged from a subject at the time of its discharge (for example, Patent Publication 3) and determining acceptability of the body metabolic function with respect to sugar based on the magnitude of the blood sugar value or urine sugar value at the time of its measurement. The reason that the measurement is made after passage of the given time after meal is that the blood sugar level or urine sugar level reaches a peak value approximately after passage of the given time after meal and the body metabolic function with respect to sugar is evaluated based on this peak value.
Patent Publication 1
   Japanese Patent Laid-Open Publication No. 2003-334180
Patent Publication 2
   Japanese Patent Laid-Open Publication No. 2003-302406
Patent Publication 3
   Japanese Patent Laid-Open Publication No. 9-297120

However, since the digestion process and the content of carbohydrates in the intestine vary according to the type of taken meal, the peak times of the blood sugar level and urine sugar level based on the taken meal and the values of the peaks vary. Further, an ability to restore the blood sugar level, i.e., glucose tolerance, differs depending on individuals. Therefore, the blood sugar level does not always reaches the peak value after passage of the given time after meal. Meanwhile, the above conventional blood sugar meter or urine sugar meter measures a blood sugar level or urine sugar level at the time of sampling or discharge thereof.

Accordingly, evaluation of the body metabolic function with respect to sugar by use of the conventional blood sugar meter lacks reliability, because a blood sugar level or urine sugar level measured by the conventional blood sugar meter after passage of a given time after meal (for example, after passage of one hour after meal in the case of the blood sugar meter) is not always a peak value.

Further, to make a highly reliable evaluation by use of the conventional blood sugar meter, a blood sugar level must be measured at short time intervals after meal (for example, at 10-minute intervals after meal) to find its changing trend and the time when the blood sugar level reaches the peak value must be specified. Thus, it was troublesome.

In view of the above circumstances, an object of the present invention is to provide a urine sugar meter which can provide highly reliable result data without much effort in evaluating a body metabolic function with respect to sugar.

US 5,420,108 A relates to a method for controlling diabetes mellitus in a diabetic patient. comprising testing the blood sugar level and the urine sugar level, and administering insulin before a meal and sugar after a meal as required by the results of the blood and urine sugar tests.

US 2003/208113 A1 relates to a system for assisting a person to maintain a blood glucose level between predetermined limits comprising an electronic apparatus, data entry means, a display, a clock, a memory, and a data processor.

### SUMMARY OF THE INVENTION

To achieve the above object, a urine sugar meter of the present invention is provided according to claim 1.

A urine sugar meter of the present invention measures an immediately-before-meal urine sugar value by immediately-before-meal urine sugar measuring means, notifies a subject that an after-meal urine sugar value should be measured after passage of a predetermined time (preferably 2 hours) by after-meal urine sugar time signal means, measures the after-meal urine sugar value by after-meal urine sugar measuring means, computes a urine sugar change value which represents the difference between the after-meal urine sugar value and the immediately-before-meal urine sugar value by urine sugar change value computing means, and outputs the urine sugar change value by urine sugar change data output means. Thus, the measurement is carried out only immediately before meal and after meal. Further, a high blood sugar level which occurs in the blood based on taken meal is reflected on a urine sugar change value obtained by measurement after passage of a predetermined time by discharging urine collected in the bladder in immediately-before-meal measurement. In other words, most of sugar which is not used in a body after meal can be secured due to an insufficient body metabolic function.

Further, after passage of two hours from measurement of the immediately-before-meal urine sugar value, sugar that is not used in the body after meal is discharged into the urine and is hardly diluted by an increase of the urine. Thus, the subject can be provided with a more reliable urine sugar change value.

Further, by determining acceptability of a change in the amount of sugar in the body by sugar amount change acceptability determining means and outputting an indicator thereabout by urine sugar change data output means, the subject can be provided with the result of determination of body metabolic function with respect to sugar without effort.

Further, by determining the acceptability of the change in the amount of sugar in the body to be "good" when the urine sugar change value is not larger than 20 mg/dl, "slightly high" when the urine sugar change value is larger than 20 mg/dl but not larger than 50 mg/dl and "high" when the urine sugar change value is larger than 50 mg/dl, the subject can be provided with a more advantageous result of determination of the body metabolic function with respect to sugar.

Further, by notifying the subject that the after-meal urine sugar value should be measured after passage of a predetermined time (preferably 1 hour) by after-meal urine sugar time signal means when the result of determination of the change in the amount of sugar in the body is unacceptable, the subject can be provided with the urine sugar change value and the result of determination of the body metabolic function with respect to sugar continuously.

Further, the point when one hour is elapsed from measurement of the after-meal urine sugar value is suited for collection of urine and evaluation intervals. Thus, the subject can be provided with the urine sugar change value and the result of determination of the body metabolic function with respect to sugar continuously without much burden.

Further, by measuring a before-meal urine sugar value by before-meal urine sugar measuring means and notifying the subject that an immediately-before-meal urine sugar value should be measured after passage of a predetermined time (preferably one hour) by immediately-before-meal urine sugar time signal means, the subject can be provided with an accurate immediately-before-meal urine sugar value.

Further, the point when one hour is elapsed from measurement of the before-meal urine sugar value is suited for collection of urine. Thus, the subject can be provided with the immediately-before-meal urine sugar value accurately without much burden.

### BRIEF DESCRIPTION OF THE DRAWINGS

Fig. 1 is an external view of a urine sugar meter according to the present invention in a detached state.
Fig. 2 is an external view of the urine sugar meter according to the present invention in a stored state.
Fig. 3 is a block diagram illustrating the constitution of the urine sugar meter according to the present invention.
Fig. 4 is a diagram illustrating the display form of the display section in Fig. 1.
Fig. 5 is a main flowchart illustrating procedures about the handling and operation of the urine sugar meter according to the present invention.
Fig. 6 is a subroutine flowchart illustrating procedures about the handling and operation of the urine sugar meter not covered by the present invention.
Fig. 7 is a diagram illustrating measuring urine in a toilet.
Fig. 8 is a diagram illustrating measuring urine in a container.

### DETAILED DESCRIPTION OF THE PREFERRED EMBODIMENTS

The urine sugar meter of the present invention has one event mode function. To achieve the event mode function, the urine sugar meter comprises before-meal urine sugar measuring means, immediately-before-meal urine sugar time signal means, immediately-before-meal urine sugar measuring means, after-meal urine sugar time signal means, after-meal urine sugar measuring means, urine sugar change value computing means, sugar amount change acceptability determining means, and urine sugar change data output means.

First, each of the means for achieving one event mode function (a function for measuring a urine sugar change value between before and after meal and determining a change in the amount of sugar in a body) will be described in detail.

The before-meal urine sugar measuring means measures a before-meal urine sugar value before measurement of an immediately-before-meal urine sugar value by the immediately-before-meal urine sugar measuring means. This is means for eliminating the influence of sugar component discharged and remaining in the urine after the last meal.

The immediately-before-meal urine sugar time signal means notifies a subject that the immediately-before-meal urine sugar value should be measured after a predetermined time is elapsed from measurement of the before-meal urine sugar value by the before-meal urine sugar measuring means. The predetermined time refers to a time suited for collecting urine to be examined until immediately before meal. The predetermined time is preferably one hour.

The immediately-before-meal urine sugar measuring means measures the immediately-before-meal urine sugar value. This is means for acquiring a urine sugar value which serves as a reference for determining a urine sugar change value.

The after-meal urine sugar time signal means notifies the subject that an after-meal urine sugar value should be measured after a predetermined time is elapsed from measurement of the immediately-before-meal urine sugar value by the immediately-before-meal urine sugar measuring means. The predetermined time refers to a time during which sugar that is not used in the body after meal is discharged into the urine and hardly diluted by an increase of the urine, i.e., a time suited for collecting urine to be examined. The predetermined time is preferably two hours.

The after-meal urine sugar measuring means measures the after-meal urine sugar value after notification by the after-meal urine sugar time signal means. This is means for acquiring a urine sugar value which is used to determine the urine sugar change value.

The urine sugar change value computing means computes, as the urine sugar change value, the difference between the after-meal urine sugar value measured by the after-meal urine sugar measuring means and the immediately-before-meal urine sugar value measured by the immediately-before-meal urine sugar measuring means. This is means for determining the amount of sugar which is not used in the body after meal due to an insufficient body metabolic function.

The sugar amount change acceptability determining means determines acceptability of a change in the amount of sugar in the body based on the urine sugar change value computed by the urine sugar change value computing means. This is means for automatically determining the amount of sugar which is not used in the body after meal due to an insufficient body metabolic function. The acceptability of the change in the amount of sugar in the body is desirably determined to be "good" when the urine sugar change value is not larger than 20 mg/dl, "slightly high" when the urine sugar change value is larger than 20 mg/dl but not larger than 50 mg/dl and "high" when the urine sugar change value is larger than 50 mg/dl. This is because it is a determination range suited for determining diseases such as diabetes and arteriosclerosis and prevention of the diseases.

The urine sugar change data output means outputs the urine sugar change value computed by the urine sugar change value computing means and an indicator about the acceptability of the change in the amount of sugar in the body which has been determined by the sugar amount change acceptability determining means. This is means for providing the subject with the results.

According to the above constitution, the urine sugar meter measures a before-meal urine sugar value by the before-meal urine sugar measuring means, notifies a subject that an immediately-before-meal urine sugar value should be measured after a predetermined time (preferably one hour) is elapsed from measurement of the before-meal urine sugar value by the immediately-before-meal urine sugar time signal means, measures the immediately-before-meal urine sugar value after the notification by the immediately-before-meal urine sugar measuring means, notifies the subject that an after-meal urine sugar value should be measured after a predetermined time (preferably two hours) is elapsed from measurement of the immediately-before-meal urine sugar value by the after-meal urine sugar time signal means, measures the after-meal urine sugar value after the notification by the after-meal urine sugar measuring means, computes a urine sugar change value which represents the difference between the after-meal urine sugar value and the immediately-before-meal urine sugar value by the urine sugar change value computing means, determines acceptability of a change in the amount of sugar in the body based on the computed urine sugar change value (preferably determines it to be "good" when the urine sugar change value is not larger than 20 mg/dl, "slightly high" when the urine sugar change value is larger than 20 mg/dl but not larger than 50 mg/dl and "high" when the urine sugar change value is larger than 50 mg/dl) by the sugar amount change acceptability determining means, outputs the urine sugar change value and an indicator about the acceptability of the change in the amount of sugar in the body by the urine sugar change data output means, and notifies the subject that the after-meal urine sugar value should be measured after a predetermined time (preferably one hour) is elapsed from measurement of the after-meal urine sugar value when the determined change in the amount of sugar in the body is not acceptable by the after-meal urine sugar time signal means. Therefore, the subject can be provided with a highly reliable urine sugar change value and a highly reliable result of determination of body metabolic function with respect to sugar accurately and without much effort.

Hereinafter, the above forms will be further described by use of, as an embodiment, a urine sugar meter which has a urine sugar sensor capable of detecting not only urine sugar of medium to high concentration range but also urine sugar of low concentration range which has been difficult to detect accurately.

### Examples

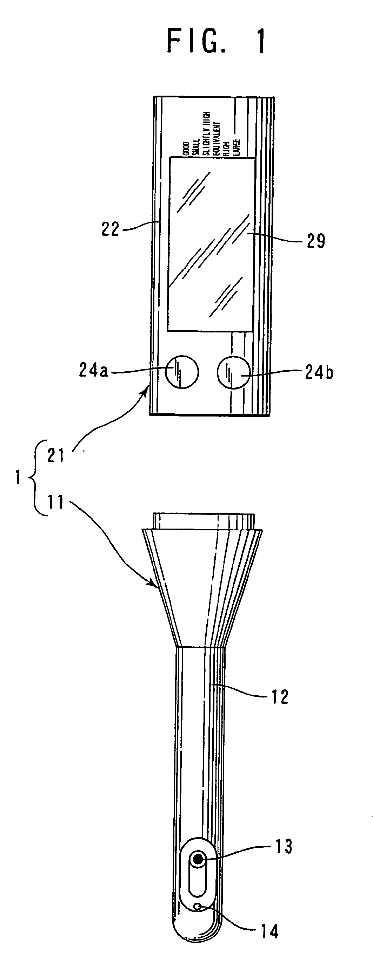

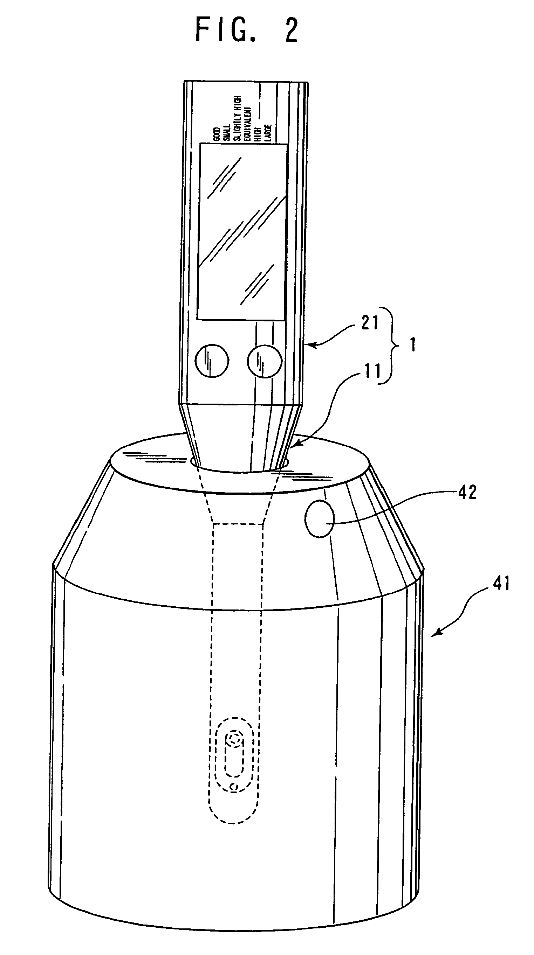

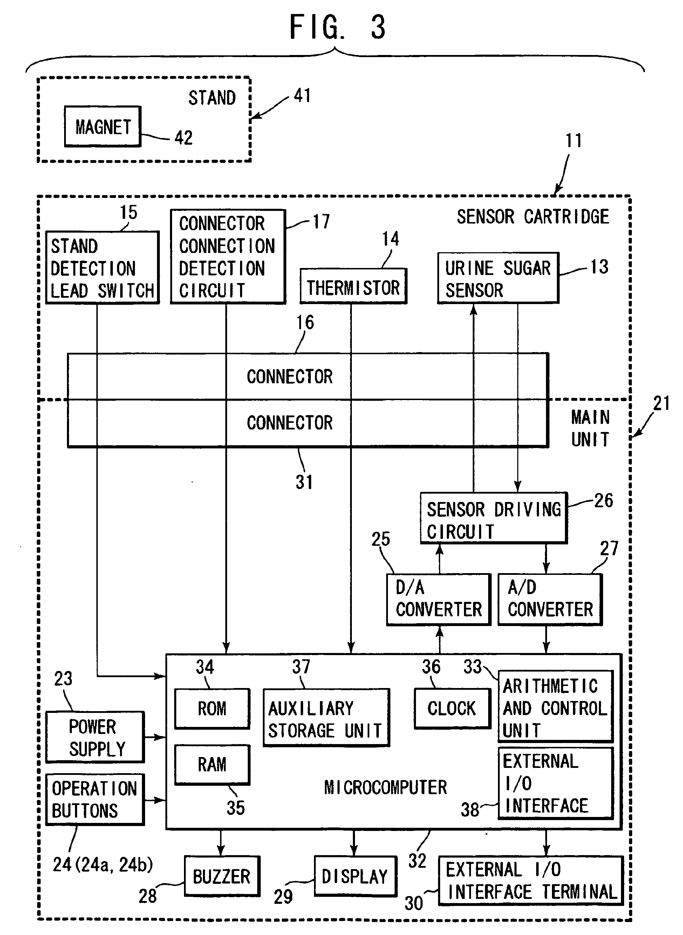

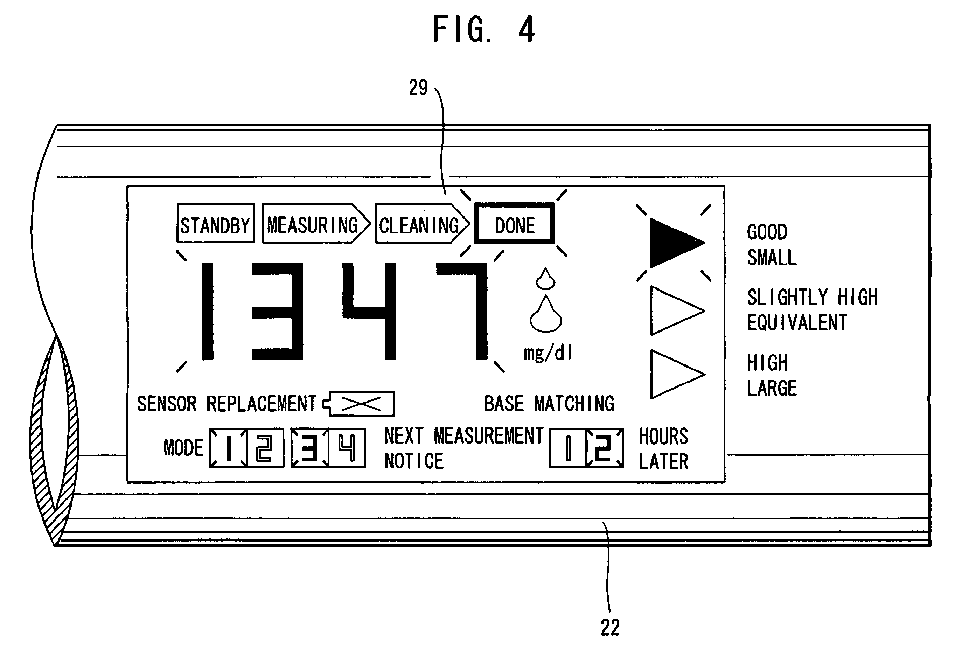

First, the constitution of urine sugar meter according to the present invention will be described in detail by use of external views shown in Figs. 1 and 2, a block diagram shown in Fig. 3 and a diagram shown in Fig. 4 that illustrates the display form of a display section.

A urine sugar meter 1 according to the present invention roughly comprises a sensor cartridge 11 that detects sugar in the urine electrochemically and a main unit 21 that serves as a grip during measurement and measures and displays the concentration of sugar in the urine based on detection made by the sensor cartridge 11. The urine sugar meter 1 according to the present invention can be inserted and stored in a stand 41 which has a magnet 42 when not used.

The sensor cartridge 11 has a chassis 12. The chassis 12 comprises a urine sugar sensor 13, a thermistor 14, a stand detection lead switch 15, a connector 16, and a connector connection detection circuit 17. Meanwhile, the main unit 21 has a chassis 22. The chassis 22 comprises a power supply 23, operation buttons 24 (24a, 24b), a D/A converter 25, a sensor driving circuit 26, an A/D converter 27, a buzzer 28, a display 29, an external I/O interface terminal 30, a connector 31, and a microcomputer 32.

The urine sugar sensor 13 detects the concentration of sugar in the urine over a wide range without the influence of foreign substances in the urine.

The thermistor 14 detects the temperature of the urine at the time of measurement and corrects the temperature characteristics of the urine sugar sensor 13.

The stand detection lead switch 15 enables mode setting, correction, and measurement of the concentration of sugar in the urine by detecting a change in the strength of a magnetic field in the magnet 42 when the urine sugar meter 1 is taken out of the stand 41.

The power supply 23 is a battery and supplies electric power to each of the sections in the electrical system of the urine sugar meter 1.

The operation buttons 24 (24a and 24b) detect being pressed and are used by a user to select, set and register a mode or a meal menu or correct a meal menu.

The D/A converter 25 converts a digital signal (driving signal) from the microcomputer 32 to an analog signal (driving signal) and outputs the analog signal.

The sensor driving circuit 26 provides a driving signal to the urine sugar sensor 13 based on the driving signal from the D/A converter 25 and inputs and outputs a detection signal detected by the urine sugar sensor 13.

The A/D converter 27 converts the analog signal (detection signal) from the sensor driving circuit 26 to a digital signal (detection signal) and outputs the digital signal.

The buzzer 28 emits a buzzer sound to notify a user of the start of measurement of urine sugar based on a signal from the microcomputer 32.

The display 29 displays the status of correction, the status of measurement, notice of replacement of the sensor, notice of replacement of the battery, the status of selected mode, notice of the next measurement, notice of urine sugar measurement results and notice of determination results, based on a signal from the microcomputer 32. More specifically, the display 29 displays the status of correction by lighting "base matching" and "droplet marks" thereabove out of display items shown in Fig. 4 at the time of correction. Further, the display 29 displays the status of measurement by lighting any of "standby", "measuring", "cleaning" and "done" in the measuring process. Further, it displays notice of replacement of the sensor cartridge 11 by lighting "sensor replacement" at the time when the urine sugar sensor 13 is to be replaced. Further, it displays notice of replacement of the battery by lighting "battery mark" at the time when the battery is to be replaced. Further, the display 29 displays the status of selected mode by lighting "mode" and "1", "2", "3" or "4" adjacent thereto that corresponds to the selected mode at the time of event. Further, the display 29 displays notice of the next measurement by lighting "next measurement notice" and "hours later", and "1" or "2" therebetween at the time of event. Further, the display 29 displays notice of urine sugar measurement results by lighting a urine sugar change value, a reference urine sugar change value or a normal urine sugar change value in "4-digit number" after measurement of each of the values. Further, it displays the status of selected mode by lighting one of three "triangle marks" which corresponds to the corresponding determination criteria printed on the chassis 22 after determination of each of the values.

The external I/O interface terminal 30 exchanges various data (i.e. input data from an external device and output data from the urine sugar meter 1 such as urine sugar measurement results and determination results) with an external device such as a personal computer, based on a signal from an external I/O interface 38 in the microcomputer 32.

The connectors 16 and 31 connect the sensor cartridge 11 and the main unit 21 to each other to enable electrical communication therebetween.

The connector connection detection circuit 17 detects whether the connector 16 and the connector 31 are in connection with each other.

The microcomputer 32 comprises an arithmetic and control unit 33, a ROM 34, a RAM 35, a clock 36, an auxiliary storage unit 37, the external I/O interface 38, and IO ports. The arithmetic and control unit 33 computes various urine sugar values and various determinations and is responsible for various controls. The ROM 34 stores programs for control and computations. The RAM 35 temporarily stores computation results, programs read from an external device, and the like. The clock 36 keeps time for various notices. The auxiliary storage unit 37 stores the selected mode, meal menus, various computed urine sugar values and determination results until they are updated. The external I/O interface 38 exchanges various data (i.e. input data from an external device and output data from the urine sugar meter 1 such as urine sugar measurement results and determination results) with an external device such as a personal computer. The IO ports are connected to the sections in the electrical system. The microcomputer 32 notifies a user that an immediately-before-meal urine sugar value, and an after-meal urine sugar value should be measured, measures a before-meal urine sugar value, the immediately-before-meal urine sugar value, and the after-meal urine sugar value, computes a urine sugar change value (difference between the after-meal urine sugar value and the immediately-before-meal urine sugar value), determines a change in sugar amount in a body based on the urine sugar change value and a normal meal menu based on the meal difference urine sugar change value, and controls outputs of the results of these computations and determinations.

The urine sugar sensor 13, D/A converter 25, sensor driving circuit 26, A/D converter 27 and microcomputer 32 constitute before-meal urine sugar measuring means, immediately-before-meal urine sugar measuring means, and after-meal urine sugar measuring means. Further, the buzzer 28 and the microcomputer 32 constitute immediately-before-meal urine sugar time signal means, and after-meal urine sugar time signal means. In addition, the microcomputer 32 constitutes urine sugar change value computing means, and sugar amount change determining means sugar change value computing means, meal difference urine sugar. Furthermore, the microcomputer 32 and the display 29 or the microcomputer 32 and the external I/O interface terminal 30 constitute urine sugar change data output means.

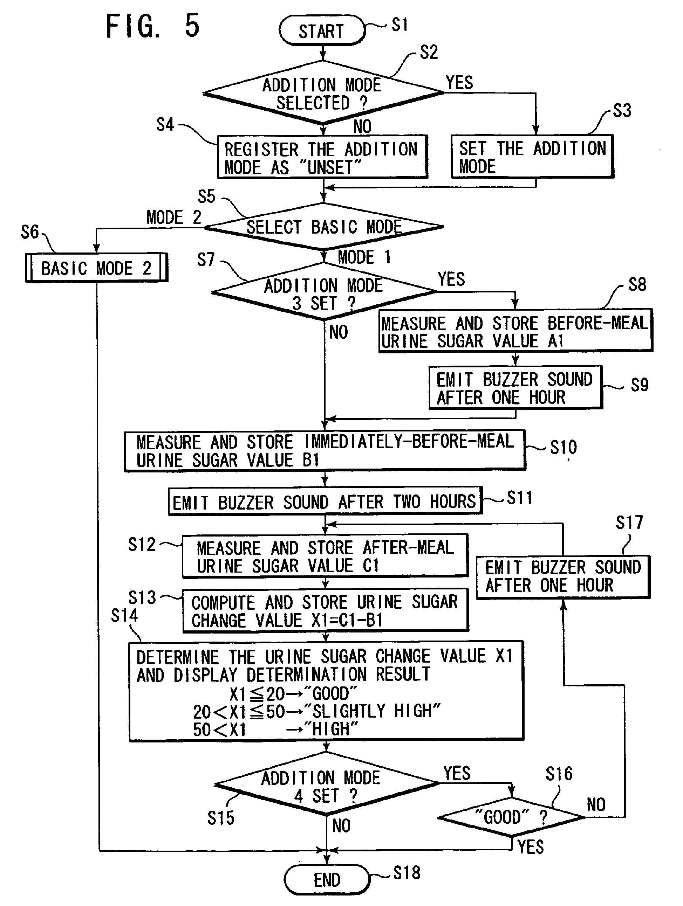

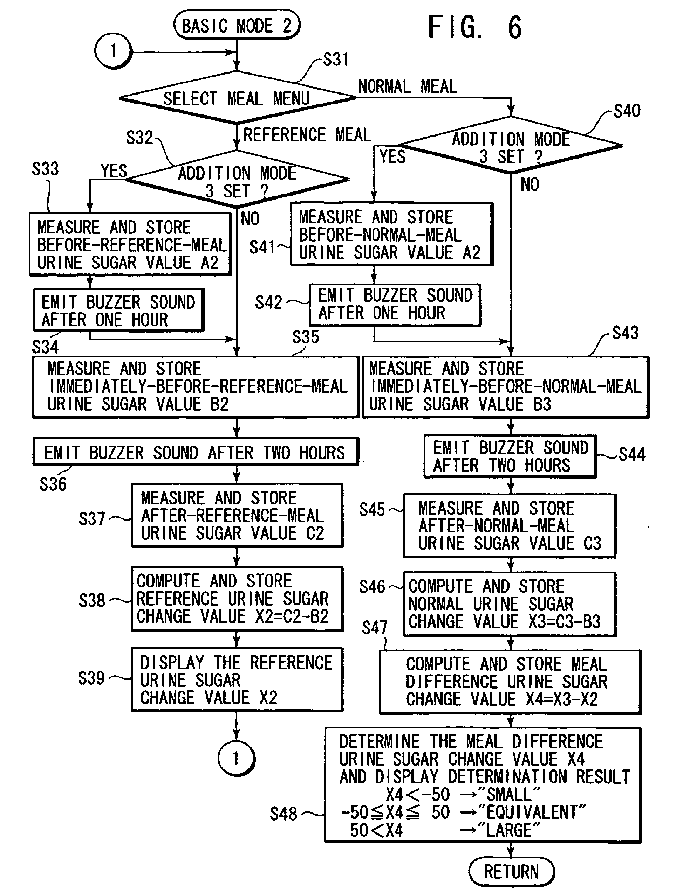

Next, the handling and operation of the urine sugar meter according to the present invention will be described by use of flowcharts shown in Figs. 5 and 6.

First, when the urine sugar meter 1 is taken out of the stand 41, the stand detection lead switch 15 is activated, and the power supply 23 supplies electric power to the sections in the electrical system, whereby the urine sugar meter 1 is activated (STEP S1).

Then, in the microcomputer 32, it is determined whether an addition mode has been selected by the operation buttons 24 (24a, 24b) (STEP S2). The addition mode refers to a mode 3 (a function for measuring an immediately-before-meal urine sugar value after measuring a before-meal urine sugar value) and a mode 4 (a function for measuring an after-meal urine sugar value again depending on the result of measurement of the after-meal urine sugar value).

Then, if the addition mode has been selected by the operation buttons 24 (24a, 24b) ("YES" in STEP S2), this selected addition mode is set and registered, and the mode "3", mode "4" or modes "3, 4" are lit and displayed on the display 29 (STEP S3). Meanwhile, if the addition mode has not been selected by the operation buttons 24 (24a, 24b) ("NO" in STEP S2), the addition mode is registered as "unset", and the modes "3" and "4" are turned off on the display 29 (STEP S4).

Then, in the microcomputer 32, it is determined whether a basic mode has been selected by the operation buttons 24 (24a, 24b) (STEP S5). The basic mode refers to a mode 1 (a function for measuring a urine sugar change value and determining acceptability of a change in the amount of sugar in a body) and a mode 2 (a function for measuring a meal difference urine sugar change value and determining acceptability of a normal meal menu) which is not covered by the present invention.

Then, if the mode 2 has been selected by the operation buttons 24 (24a, 24b) ("mode 2" in STEP S5), the selected mode 2 is set and registered, the mode "2" is lit and displayed on the display 29, and the microcomputer 32 proceeds to STEP S6 which will be described later. Meanwhile, if the mode 1 has been selected by the operation buttons 24 (24a, 24b) ("mode 1" in STEP S5), the selected mode 1 is set and registered, the mode "1" is lit and displayed on the display 29, and it is then determined in the microcomputer 32 whether the mode 3 has been set (STEP S7).

Then, if the mode 3 has been set ("YES" in STEP S7), a sugar component in the urine is detected by the urine sugar sensor 13, a detection signal which has passed through the sensor driving circuit 26 and the A/D converter 27 is taken in by the microcomputer 32, this detection signal is computed as a before-meal urine sugar value A1 by the arithmetic and control unit 33, this before-meal urine sugar value A1 is stored in the auxiliary storage unit 37, and the next measurement notice "1" hour later is lit and displayed on the display 29 (STEP S8). Then, after one hour is timed by the clock 36, the buzzer 28 emits a buzzer sound to notify a subject that immediately-before-meal urine sugar will be measured (STEP S9).

Meanwhile, if the mode 3 has not been set ("NO" in STEP S7) or after STEP S9, a sugar component in the urine is detected by the urine sugar sensor 13, a detection signal which has passed through the sensor driving circuit 26 and the A/D converter 27 is taken in by the microcomputer 32, this detection signal is computed as an immediately-before-meal urine sugar value B1 by the arithmetic and control unit 33, this immediately-before-meal urine sugar value B1 is stored in the auxiliary storage unit 37, and the next measurement notice "2" hours later is lit and displayed on the display 29 (STEP S10). Immediately after measurement of the immediately-before-meal urine sugar value B1, the subject has a meal. Then, after two hours are timed by the clock 36, the buzzer 28 emits a buzzer sound to notify the subject that after-meal urine sugar will be measured (STEP S11).

Then, a sugar component in the urine is detected by the urine sugar sensor 13, a detection signal which has passed through the sensor driving circuit 26 and the A/D converter 27 is taken in by the microcomputer 32, this detection signal is computed as an after-meal urine sugar value C1 by the arithmetic and control unit 33, and this after-meal urine sugar value C1 is stored in the auxiliary storage unit 37 (STEP S12).

Then, the difference between the after-meal urine sugar value C1 and the immediately-before-meal urine sugar value B1 is computed as a urine sugar change value X1 by the arithmetic and control unit 33, and this urine sugar change value X1 is stored in the auxiliary storage unit 37 (STEP S13).

Then, in the arithmetic and control unit 33, the computed urine sugar change value X1 is determined to be "good" when it is not larger than 20 mg/dl, "slightly high" when larger than 20 mg/dl but not larger than 50 mg/dl and "high" when larger than 50 mg/dl, and this determination result is stored in the auxiliary storage unit 37. Then, the urine sugar change value X1 and the determination result (i.e. a "triangle mark" corresponding to the corresponding determination criteria printed on the chassis 22) are displayed on the display 29 and output by the external I/O interface terminal 30 (STEP S14).

Then, in the microcomputer 32, it is determined whether the mode 4 has been set (STEP S15). Then, if the mode 4 has been set ("YES" in STEP S15), it is determined whether the foregoing determination result is "good" (STEP S16). Then, if the foregoing determination result is not "good" ("NO" in STEP S16), the next measurement notice "1" hour later is lit and displayed on the display 29, the buzzer 28 emits a buzzer sound to notify the subject that the after-meal urine sugar will be measured again after one hour is timed by the clock 36 (STEP S17), and the microcomputer returns to STEP S12 to repeat the steps.

Meanwhile, if the mode 4 has not been set ("NO" in STEP S15) or if the foregoing determination result is "good" ("YES" in STEP S16), this series of steps are ended (STEP S18).

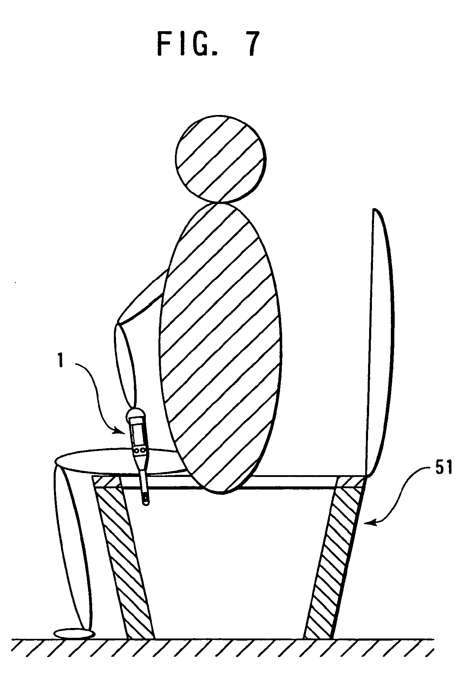

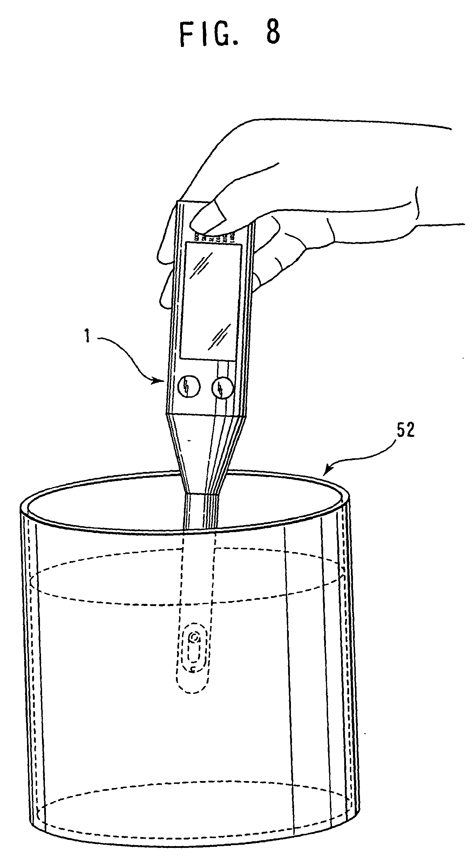

The above before-meal, immediately-before-meal, and after-meal measurements are carried out in such a manner that a subject holds the urine sugar meter 1 in a hand and applies urine to the urine sugar sensor 13 portion in a toilet 51 as shown in Fig. 7 or that urine is collected in a container 52, and a subject holds the urine sugar meter 1 in a hand and immerses the urine sugar sensor 13 portion in the urine as shown in Fig. 8.

An embodiment of the urine sugar meter according to the present invention has been specifically described above. Alternatively, the urine sugar meter of the present invention may be constituted that meal data is input by the operation buttons, stored continuously in the auxiliary storage unit 37 and later retrieved or searched as texts or images.

## Claims

1. A urine sugar meter comprising:
immediately-before-meal urine sugar measuring means (32),
after-meal urine sugar time signal means,
after-meal urine sugar measuring means,
urine sugar change value computing means (32), and
urine sugar change data output means,
**characterized in that**,
the urine sugar meter further comprising,
sugar amount change acceptability determining means (33),
wherein
the immediately-before-meal urine sugar measuring means (32) is adapted to measure an immediately-before-meal urine sugar value,
the after-meal urine sugar time signal means is adapted to notify a subject that an after-meal urine sugar value should be measured after passage of a predetermined time from measurement of the immediately-before-meal urine sugar value by the immediately-before-meal urine sugar measuring means,
the after-meal urine sugar measuring means is adapted to measure the after-meal urine sugar value after notification by the after-meal urine sugar time signal means,
the urine sugar change value computing means (32) is adapted to compute the difference between the after-meal urine sugar value measured by the after-meal urine sugar measuring means and the immediately-before-meal urine sugar value measured by the immediately-before-meal urine sugar measuring means as a urine sugar change value,
the urine sugar change data output means is adapted to output the urine sugar change value computed by the urine sugar change value computing means,
the sugar amount change acceptability determining means (33) is adapted to determine acceptability of a change in the amount of sugar in a body based on the urine sugar change value computed by the urine sugar change value computing means, and
the sugar amount change acceptability determining means is adapted to determine that the change in the amount of sugar in the body is "good" which means "acceptable" when the urine sugar change value is not larger than 20 mg/dl, "slightly high" which means "unacceptable" when the urine sugar change value is larger than 20 mg/dl but not larger than 50 mg/dl and "high" which means "unacceptable" when the urine sugar change value is larger than 50 mg/dl.

2. The urine sugar meter of claim 1, wherein the predetermined time elapsed from measurement of the immediately-before-meal urine sugar value is two hours.

3. The urine sugar meter of claim 1 or 2, wherein
the urine sugar change data output means is also adapted to output an indicator about the acceptability of the change in the amount of sugar in the body determined by the sugar amount change acceptability determining means.

4. The urine sugar meter of claim 1, wherein the after-meal urine sugar time signal means is also adapted to notify the subject that the after-meal urine sugar value should be remeasured after passage of a predetermined time from measurement of the after-meal urine sugar value by the after-meal urine sugar measuring means, when the change in the amount of sugar in the body which has been determined by the sugar amount change acceptability determining means (33) is unacceptable.

5. The urine sugar meter of claim 4, wherein the predetermined time elapsed from measurement of the after-meal urine sugar value is one hour.

6. The urine sugar meter of any one of claims 1 to 5, further comprising:
before-meal urine sugar measuring means, and
immediately-before-meal urine sugar time signal means,
wherein
the before-meal urine sugar measuring means is adapted to measure a before-meal urine sugar value before measurement of the immediately-before-meal urine sugar value by the immediately-before-meal urine sugar measuring means, and
the immediately-before-meal urine sugar time signal means is adapted to notify the subject that the immediately-before-meal urine sugar value should be measured after passage of a predetermined time from measurement of the before-meal urine sugar value by the before-meal urine sugar measuring means.

7. The urine sugar meter of claim 6, wherein the predetermined time elapsed from measurement of the before-meal urine sugar value is one hour.

## Patentansprüche

1. Urinzuckermessgerät, umfassend:
Unmittelbar-vor-der-Mahlzeit-Urinzuckermessmittel (32),
Nach-der-Mahlzeit-Urinzuckerzeitsignalmittel,
Nach-der-Mahlzeit-Urinzuckermessmittel,
Urinzuckeränderungswertberechnungsmittel (32) und
Urinzuckeränderungsdatenausgabemittel,
**dadurch gekennzeichnet, dass**
das Urinzuckermessgerät des Weiteren umfasst:
Zuckermengenänderungsannehmbarkeitsbestimmungsmittel (33),
wobei
das Unmittelbar-vor-der-Mahlzeit-Urinzuckermessmittel (32) dafür ausgelegt ist, einen Unmittelbar-vor-der-Mahlzeit-Urinzuckerwert zu messen,
das Nach-der-Mahlzeit-Urinzuckerzeitsignalmittel dafür ausgelegt ist, einen Betroffenen darüber zu benachrichtigen, dass ein Nach-der-Mahlzeit-Urinzuckerwert nach Verstreichen einer vorbestimmten Zeit ab der Messung des Unmittelbar-vor-der-Mahlzeit-Urinzuckerwertes durch das Unmittelbar-vor-der-Mahlzeit-Urinzuckermessmittel gemessen werden soll,
das Nach-der-Mahlzeit-Urinzuckermessmittel dafür ausgelegt ist, den Nach-der-Mahlzeit-Urinzuckerwert nach Benachrichtigung durch das Nach-der-Mahlzeit-Urinzuckerzeitsignalmittel zu messen,
das Urinzuckeränderungswertberechnungsmittel (32) dafür ausgelegt ist, die Differenz zwischen dem Nach-der-Mahlzeit-Urinzuckerwert aus der Messung durch das Nach-der-Mahlzeit-Urinzuckermessmittel und dem Unmittelbar-vor-der-Mahlzeit-Urinzuckerwert aus der Messung durch das Unmittelbar-vor-der-Mahlzeit-Urinzuckermessmittel als Urinzuckeränderungswert zu berechnen,
das Urinzuckeränderungsdatenausgabemittel dafür ausgelegt ist, den Urinzuckeränderungswert aus der Berechnung durch das Urinzuckeränderungswertberechnungsmittel auszugeben,
das Zuckermengenänderungsannehmbarkeitsbestimmungsmittel (33) dafür ausgelegt ist, eine Annehmbarkeit einer Änderung der Menge an Zucker in einem Körper auf Grundlage des Urinzuckeränderungswertes aus der Berechnung durch das Urinzuckeränderungswertberechnungsmittel zu bestimmen, und
das Zuckermengenänderungsannehmbarkeitsbestimmungsmittel dafür ausgelegt ist zu bestimmen, dass die Änderung der Menge an Zucker in dem Körper "gut" und mithin "annehmbar" ist, wenn der Urinzuckeränderungswert nicht größer als 20 mg/dl ist,
"leicht erhöht" und mithin "nicht annehmbar" ist, wenn der Urinzuckeränderungswert größer als 20 mg/dl und nicht größer als 50 mg/dl ist, und "erhöht" und mithin "nicht annehmbar" ist, wenn der Urinzuckeränderungswert größer als 50 mg/dl ist.

2. Urinzuckermessgerät nach Anspruch 1, wobei die ab der Messung des Unmittelbar-vor-der-Mahlzeit-Urinzuckerwertes verstrichene vorbestimmte Zeit zwei Stunden ist.

3. Urinzuckermessgerät nach Anspruch 1 oder 2, wobei:
das Urinzuckeränderungsdatenausgabemittel dafür ausgelegt ist, eine Angabe hinsichtlich der Annehmbarkeit der Änderung der Menge an Zucker in dem Körper aus der Bestimmung durch das Zuckermengenänderungsannehmbarkeitsbestimmungsmittel auszugeben.

4. Urinzuckermessgerät nach Anspruch 1, wobei das Nach-der-Mahlzeit-Urinzuckerzeitsignalmittel dafür ausgelegt ist, den Betroffenen darüber zu benachrichtigen, dass der Nach-der-Mahlzeit-Urinzuckerwert nach Verstreichen einer vorbestimmten Zeit ab der Messung des Nach-der-Mahlzeit-Urinzuckerwertes durch das Nach-der-Mahlzeit-Urinzuckermessmittel erneut gemessen werden soll, wenn die Änderung der Menge an Zucker in dem Körper gemäß Bestimmung durch das Zuckermengenänderungsannehmbarkeitsbestimmungsmittel (33) nicht annehmbar ist.

5. Urinzuckermessgerät nach Anspruch 4, wobei die ab der Messung des Nach-der-Mahlzeit-Urinzuckerwertes verstrichene vorbestimmte Zeit eine Stunde ist.

6. Urinzuckermessgerät nach einem der Ansprüche 1 bis 5, des Weiteren umfassend:
Vor-der-Mahlzeit-Urinzuckermessmittel und
Unmittelbar-vor-der-Mahlzeit-Urinzuckerzeitsignalmittel,
wobei
das Vor-der-Mahlzeit-Urinzuckermessmittel dafür ausgelegt ist, einen Vor-der-Mahlzeit-Urinzuckerwert vor der Messung des Unmittelbar-vor-der-Mahlzeit-Urinzuckerwertes durch das Unmittelbar-vor-der-Mahlzeit-Urinzuckermessmittel zu messen, und
das Unmittelbar-vor-der-Mahlzeit-Urinzuckerzeitsignalmittel dafür ausgelegt ist, den Betroffenen darüber zu benachrichtigen, dass der Unmittelbar-vor-der-Mahlzeit-Urinzuckerwert nach Verstreichen einer vorbestimmten Zeit ab der Messung des Vor-der-Mahlzeit-Urinzuckerwertes durch das Vor-der-Mahlzeit-Urinzuckermessmittel gemessen werden soll.

7. Urinzuckermessgerät nach Anspruch 6, wobei die ab der Messung des Vor-der-Mahlzeit-Urinzuckerwertes verstrichene vorbestimmte Zeit eine Stunde ist.

## Revendications

1. Dispositif de mesure du taux de sucre dans l'urine, comprenant :
des moyens de mesure du taux de sucre dans l'urine immédiatement avant le repas (32),
des moyens de signal de temps du taux de sucre dans l'urine après le repas,
des moyens de mesure du taux de sucre dans l'urine après le repas,
des moyens de calcul de valeur de changement du taux de sucre dans l'urine (32), et
des moyens de production de données de changement du taux de sucre dans l'urine,
**caractérisé en ce que** :
le dispositif de mesure du taux de sucre dans l'urine comprend :
des moyens de détermination d'acceptabilité de changement de quantité de sucre (33),
dans lequel :
les moyens de mesure du taux de sucre dans l'urine immédiatement avant le repas (32) sont adaptés pour mesurer une valeur du taux de sucre dans l'urine immédiatement avant le repas,
les moyens de signal de temps du taux de sucre dans l'urine après le repas sont adaptés pour avertir un patient qu'une valeur du taux de sucre dans l'urine après le repas doit être mesurée après l'écoulement d'un temps prédéterminé à partir de la mesure de la valeur du taux de sucre dans l'urine immédiatement avant le repas par les moyens de mesure du taux de sucre dans l'urine immédiatement avant le repas,
les moyens de mesure du taux de sucre dans l'urine après le repas sont adaptés pour mesurer la valeur du taux de sucre dans l'urine après le repas après la notification par les moyens de signal de temps du taux de sucre dans l'urine après le repas,
les moyens de calcul de valeur de changement du taux de sucre dans l'urine (32) sont adaptés pour calculer une différence entre la valeur du taux de sucre dans l'urine après le repas par les moyens de mesure du taux de sucre dans l'urine après le repas et la valeur du taux de sucre dans l'urine immédiatement avant le repas mesurée par les moyens de mesure du taux de sucre dans l'urine immédiatement avant le repas en tant que valeur de changement du taux de sucre dans l'urine,
les moyens de production de données de changement du taux de sucre dans l'urine sont adaptés pour produire la valeur de changement du taux de sucre dans l'urine calculée par les moyens de calcul de valeur de changement du taux de sucre dans l'urine,
les moyens de détermination d'acceptabilité de changement de quantité de sucre (33) sont adaptés pour déterminer l'acceptabilité d'un changement de la quantité de sucre dans un corps en fonction de la valeur de changement du taux de sucre dans l'urine calculée par les moyens de calcul de valeur de changement du taux de sucre dans l'urine, et
les moyens de détermination d'acceptabilité de changement de quantité de sucre sont adaptés pour déterminer que le changement de la quantité de sucre dans le corps est « bon » ce qui signifie « acceptable » lorsque la valeur de changement du taux de sucre dans l'urine n'est pas supérieure à 20 mg/dl, « légèrement élevée » ce qui signifie « inacceptable » lorsque la valeur de changement du taux de sucre dans l'urine est supérieure à 20 mg/dl mais non supérieure à 50 mg/dl et « élevée » ce qui signifie « inacceptable » lorsque la valeur de changement du taux de sucre dans l'urine est supérieure à 50 mg/dl.

2. Dispositif de mesure du taux de sucre dans l'urine selon la revendication 1, dans lequel le temps prédéterminé qui s'est écoulé depuis la mesure de la valeur du taux de sucre dans l'urine immédiatement avant le repas est de deux heures.

3. Dispositif de mesure du taux de sucre dans l'urine selon la revendication 1 ou 2, dans lequel :
les moyens de production de données de changement du taux de sucre dans l'urine sont également adaptés pour produire un indicateur concernant l'acceptabilité du changement de la quantité de sucre dans le corps déterminé par les moyens de détermination d'acceptabilité de changement de quantité de sucre.

4. Dispositif de mesure du taux de sucre dans l'urine selon la revendication 1, dans lequel les moyens de signal de temps du taux de sucre dans l'urine après le repas sont également adaptés pour avertir le patient que la valeur du taux de sucre dans l'urine après le repas doit être remesurée après l'écoulement d'un temps prédéterminé depuis la mesure de la valeur du taux de sucre dans l'urine après le repas par les moyens de mesure du taux de sucre dans l'urine après le repas, lorsque le changement de quantité de sucre dans le corps qui a été déterminé par les moyens de détermination d'acceptabilité de changement de quantité de sucre (33) est inacceptable.

5. Dispositif de mesure du taux de sucre dans l'urine selon la revendication 4, dans lequel le temps prédéterminé qui s'est écoulé depuis la mesure de la valeur du taux de sucre dans l'urine après le repas est d'une heure.

6. Dispositif de mesure du taux de sucre dans l'urine selon l'une quelconque des revendications 1 à 5, comprenant en outre :
des moyens de mesure du taux de sucre dans l'urine avant le repas, et
des moyens de signal de temps du taux de sucre dans l'urine immédiatement avant le repas,
dans lequel :
les moyens de mesure du taux de sucre dans l'urine avant le repas sont adaptés pour mesurer une valeur du taux de sucre dans l'urine avant le repas, avant la mesure de la valeur du taux de sucre dans l'urine immédiatement avant le repas par les moyens de mesure du taux de sucre dans l'urine immédiatement avant le repas, et
les moyens de signal de temps du taux de sucre dans l'urine immédiatement avant le repas sont adaptés pour avertir le patient que la valeur du taux de sucre dans l'urine immédiatement avant le repas doit être mesurée après l'écoulement d'un temps prédéterminé depuis la mesure de la valeur du taux de sucre dans l'urine après le repas par les moyens de mesure du taux de sucre dans l'urine après le repas.

7. Dispositif de mesure du taux de sucre dans l'urine selon la revendication 6, dans lequel le temps prédéterminé qui s'est écoulé à partir de la mesure de la valeur du taux de sucre dans l'urine après le repas est d'une heure.
